# EUROPEAN PATENT SPECIFICATION

(11) **EP 3 419 914 B1**
(45) Date of publication and mention of the grant of the patent: **25.12.2019**
(21) Application number: 17716616.2
(22) Date of filing: 24.02.2017
(51) Int. Cl.: B65D 81/32, A61B 10/00, B01L 3/00, B65D 51/28, G01N 21/03

(54) **CONTAINER FOR BIOLOGICAL TISSUE SAMPLES**
BEHÄLTER FÜR BIOLOGISCHE GEWEBEPROBEN
CONTENANT POUR ÉCHANTILLONS DE TISSUS BIOLOGIQUES

(30) Priority: 26.02.2016 IT UB20161093
(43) Date of publication of application: 02.01.2019
(73) Proprietor: CO.ME.Plast di Costalunga Francesco Davide & Figli S.N.C., 36015 Schio (IT)
(72) Inventor: COSTALUNGA, Daria, 36100 Vicenza (IT); COSTALUNGA, Carlo, 36015 Schio (IT); COSTALUNGA, Francesco Davide, 36015 Schio (IT)
(74) Representative: Modiano, Micaela Nadia
(86) International application number: PCT/IB2017/051062
(87) International publication number: WO 2017/145102

(56) References cited:
- WO-A1-2007/006414
- JP-A- 2005 231 692
- US-A1- 2008 314 775
- US-A1- 2010 301 002
- US-A1- 2015 037 830

## Description

The present invention relates to a container for biological tissue samples.

In the field of medical analyses, it is currently known to preserve biological tissue samples to be analyzed in formaldehyde solutions (formaline).

Formaldehyde has recently passed from the "suspected of causing cancer" classification to "may cause cancer" in European Union regulations.

This has emphasized the safety problem for analyst operators, in view of the current impossibility to replace formaldehyde with another less potentially problematic preservative.

Containers for biological tissue samples have been provided and have been commercially available for a few years which, despite providing for the use of formaldehyde, seek to keep it isolated from contact with the operators.

EP2720617A1 discloses a container assembly which comprises a container for depositing a tissue sample which is provided with a bottom and a lid.

The lid comprises an upper element with a receptacle for a preservative, a gasket for sealing the receptacle and a sharp piercing element which acts as a tool to break the gasket by moving said piercing element.

The container has a first position and a second position, in which in the first position the container is isolated from the receptacle, and in the second position fluid communication is provided between the receptacle and the container.

The container comprises a separator which is arranged between the gasket and the bottom of the container and the separator is provided with at least one opening which is adapted to provide a fluid passage between the receptacle and the container.

In other known cases, such as for example in WO2007/068094A1, there are one or more blades inside the vessel, which act as tools for cutting the gasket during the closure of the lid onto the vessel in order to make the preservative flow into the vessel.

In the case of WO2003/031064A1, the container is composed, from the bottom upward, of a vessel with preservative and sealing gasket, a support with a lower basket for the tissue sample, and a closure lid.

The support for the tissue sample has, on the side of the vessel, blades which act as tools for cutting the gasket and allowing access of the basket into the preservative.

Containers of this type, however, are characterized by a certain structural complication, which affects negatively the cost of the product and leads to awkward maneuverability during use. Document US2015/037830 discloses a container in accordance with the preamble of claim 1.

The aim of the present invention is to provide a container for tissue samples that overcomes the drawbacks noted above in the background art.

Within this aim, an object is to minimize the number of components.

Another object is to maintain high safety in use.

Another object is to provide a container that has a low cost.

This aim and these and other objects, which will become better apparent hereinafter, are achieved by a container for biological tissue samples, comprising a vessel for depositing a sample of tissue and a lid adapted to engage said vessel, said lid comprising a receptacle adapted to contain a preservative and a gasket sealing the inside of said receptacle in order to separate the content from the outside, and said gasket having at least one region with a stress raiser adapted to interact with at least one corresponding portion of said vessel upon the closure, thereon, of said lid, to at least partially break said region and connect said receptacle to said vessel.

Further characteristics and advantages of the invention will become better apparent from the detailed description of a particular but not exclusive embodiment thereof, illustrated by way of nonlimiting example in the accompanying drawings, wherein:

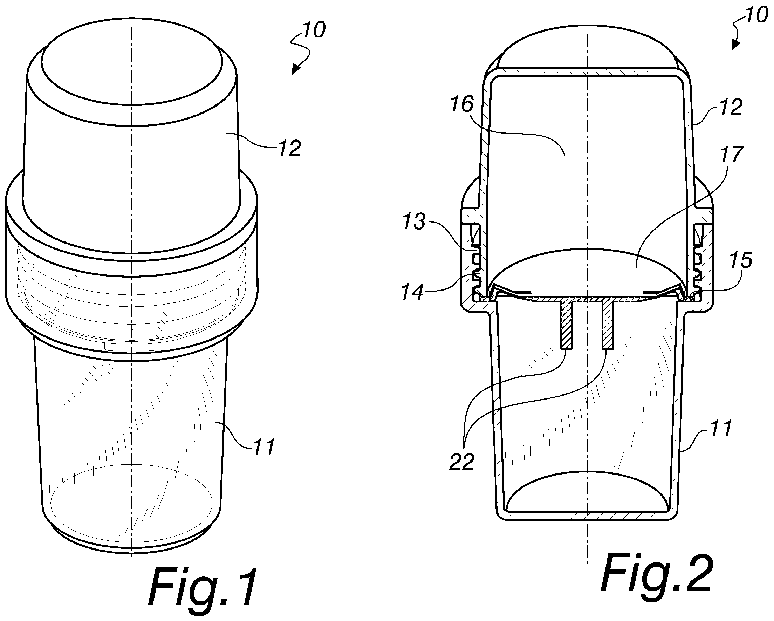

Figure 1 is a perspective view of a container according to the invention in the closed configuration;
Figure 2 is a sectional view, taken along a diametrical plane, of the container of Figure 1 in the configuration for use;

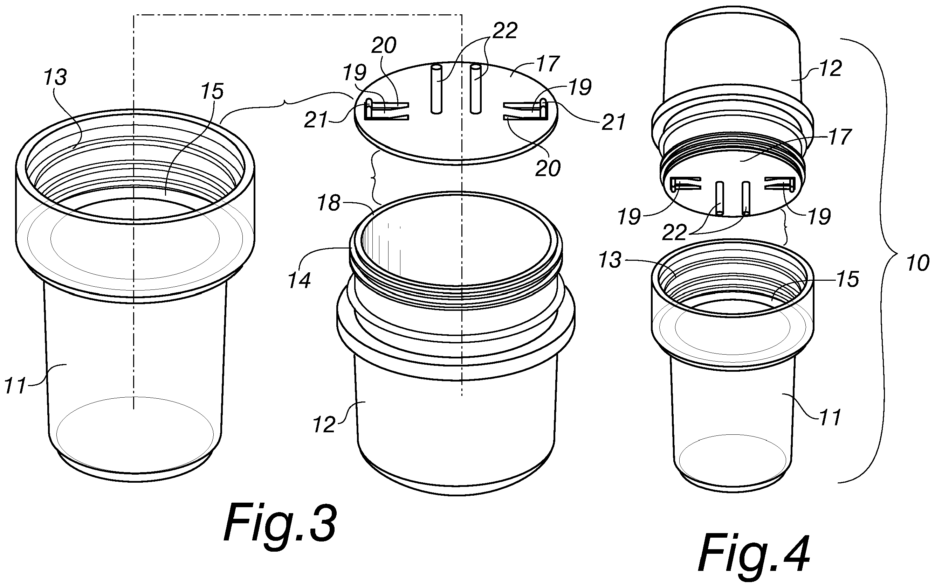

Figure 3 is an exploded perspective view of the container according to the invention;
Figure 4 is a perspective view of the container as it appears before use;
Figure 5 is an enlarged-scale view of a detail of the sectional view of Figure 2 during the screwing of the lid onto the vessel;
Figure 6 is an enlarged-scale view of a detail of the sectional view of Figure 2 at the end of the screwing of the lid onto the vessel;

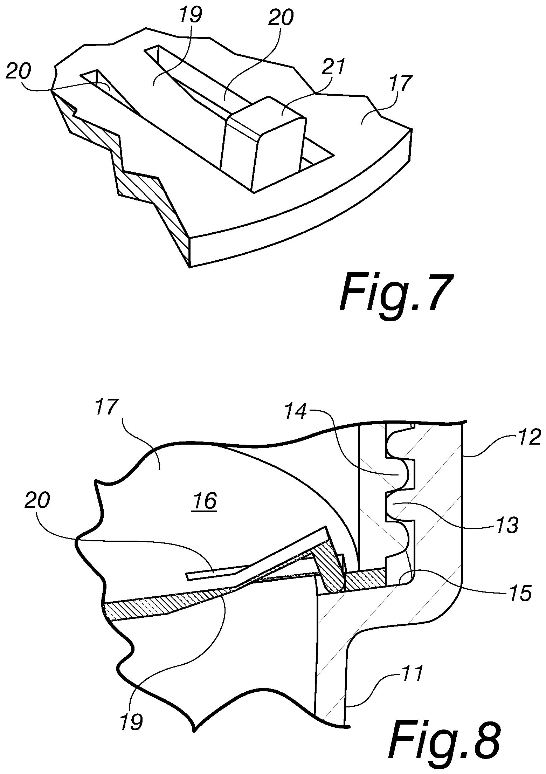

Figure 7 is a perspective view of the detail of Figures 5 and 6;
Figure 8 is a sectional perspective view that corresponds to Figure 6.

In the exemplary embodiments that follow, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

With reference to the figures, the numeral 10 designates a container for biological tissue samples.

The container 10 comprises a vessel 11 for depositing a sample of tissue and a lid 12 adapted to engage the vessel 11.

For this reason, the vessel 11 is provided in an upper region with an inner thread 13 and the lid is provided with a complementarily shaped external thread 14.

The threaded portion of the vessel 11 conveniently has a larger diameter than the rest, so as to form a step and a planar annular surface 15.

The vessel 11 and the lid 12 are conveniently provided by molding plastic material.

The lid 12 comprises a receptacle 16 which is adapted to contain a preservative and a gasket 17 which seals the interior of the receptacle 16 in order to separate the content from the outside.

The gasket 17 is conveniently constituted by a planar partition of suitable material, for example made of plastic material suitable for heat-sealing on the rim 18 of the opening of the lid 12.

Different materials, even multilayer ones, may also be provided, and the types of seal also may be different.

According to the invention, the gasket 17 is provided with two diametrically mutually opposite regions 19 which are at least partially surrounded by cross-section reduction lines 20 so as to form stress raisers (naturally, without passing through, in order to avoid compromising the seal).

Within each region 19, a raised portion 21 (shaped like a pivot) protrudes from it and is arranged so as to interact with the planar annular surface 15 of the vessel 11 when the lid 12 closes onto it, in order to break at least partially the region 19 and connect the receptacle 16 to the vessel 11.

Advantageously, each region 19 is tab-shaped and rises by flexing from the rest of the gasket 17, separating from it, as the lid 12 is screwed on and each raised portion 21 remains stationary for its resting on the annular surface 15.

In the central position of the gasket 17 there is a pair of mutually parallel prongs 22, which are adapted to constitute a sort of fork in order to facilitate the separation of the biological tissue sample for example from grip tweezers (not shown), while it is introduced in the vessel.

The container is composed of only three elements:
- vessel 11,
- lid 12,
- gasket 17.

The lid 12 is filled with preservative, for example formaldehyde, and is closed with the gasket 17, for example by high-frequency welding.

The preservative is therefore inside the receptacle 16, separated hermetically from the outside environment, and cannot come into contact with the operator.

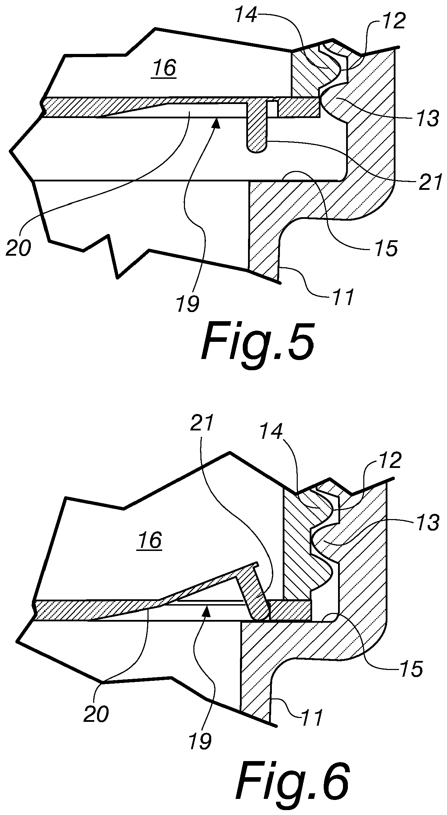

After introducing the tissue sample in the vessel 11, as mentioned for example with tweezers (not shown), the lid 12 is closed by screwing it onto the vessel 11 (Figure 5).

During screwing, at a certain point the raised portions 21 are stopped axially by contact with the annular surface 15, the gasket 17 is broken in the stress raiser regions 19 and the preservative flows into the vessel 11, remaining separate from contact with the operator.

With the complete closure of the container 10 (Figure 6), the sample is immersed in the preservative, which has always remained separate from the outside environment.

In practice it has been found that the intended aim and objects of the present invention have been achieved.

The devised container in fact overcomes the drawbacks of the background art and minimizes the number of components while maintaining high safety in use.

Since the components preferably can be made of the same plastic material, for example recyclable and/or biodegradable polymer, disposal problems are also avoided.

It should be noted that since the gasket 17 is already preset with regions 19 with stress raisers, the existence of piercing or blade-like tools to open the gasket 17 is no longer necessary but a pushing action is simply sufficient and is obtained by means of the interaction of the raised portions 21 with the annular surface 15.

Finally, the container has a low cost.

Of course, the invention is susceptible of numerous modifications and variations, all of which are within the scope of the appended claims.

Thus, for example, pusher means which are equivalent to the raised portions 21 may extend from the vessel 11 instead of from the gasket 17.

The materials used, as well as the dimensions that constitute the individual components of the invention, may be more pertinent according to the specific requirements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A container (10) for biological tissue samples, comprising a vessel (11) for depositing a sample of tissue and a lid (12) adapted to engage said vessel (11), said lid comprising a receptacle (16) adapted to contain a preservative and a gasket (17) sealing the inside of said receptacle (16) in order to separate the content from the outside, said container (10) being **characterized in that** said gasket (17) has at least one region (19) with a stress raiser adapted to interact with at least one corresponding portion of said vessel (11) upon the closure, thereon, of said lid (12), to at least partially break said region (19) and connect said receptacle (16) to said vessel (11).

2. The container according to claim 1, **characterized in that** said vessel (11) has, in an upper region, an inner thread (13) and said lid has a complementarily shaped external thread (14).

3. The container according to one or more of the preceding claims, **characterized in that** said threaded portion of said vessel (11) has a larger diameter than the rest of said vessel (11) so as to form a step and a planar annular surface (15).

4. The container according to one or more of the preceding claims, **characterized in that** said gasket (17) is constituted by a partition adapted for heat-sealing on the rim (18) of the opening of said lid (12).

5. The container according to one or more of the preceding claims, **characterized in that** said gasket (17) has two diametrically opposite regions (19) that are at least partially surrounded by cross-section reduction lines (20) so as to form stress raisers.

6. The container according to one or more of the preceding claims, **characterized in that** inside said region (19), a raised portion (21) extends therefrom which is located so as to interact with said planar annular surface (15) of said vessel (11) upon the closure, thereon, of said lid (12), in order to at least partially break said region (19) and connect said receptacle (16) to said vessel (11).

7. The container according to one or more of the preceding claims, **characterized in that** said region (19) is tab-shaped and rises by flexing from the rest of said gasket (17) as said lid (12) is screwed on and said raised portion (21) remains stationary to rest on said annular surface (15).

8. The container according to one or more of the preceding claims, **characterized in that** a pair of mutually parallel prongs (22) is extended in the central position of said gasket (17).

9. The container according to one or more of the preceding claims, **characterized in that** said vessel (11), said lid (12) and said gasket (17) are made of molded plastic material.

10. The container according to one or more of the preceding claims, **characterized in that** said vessel (11), said lid (12) and said gasket (17) are made of recyclable and/or biodegradable polymer.

## Patentansprüche

1. Ein Behälter (10) für biologische Gewebeproben, umfassend ein Gefäß (11) zum Ablegen einer Gewebeprobe und einen Deckel (12), angepasst um in das genannte Gefäß (11) einzugreifen, wobei der genannte Deckel ein Behältnis (16) umfasst, angepasst um ein Konservierungsmittel zu enthalten, und eine Dichtung (17), die das Innere des genannten Behältnisses (16) abdichtet, um den Inhalt von der Umgebung abzutrennen, wobei der genannte Behälter (10) **dadurch gekennzeichnet ist, dass** die genannte Dichtung (17) mindestens einen Bereich (19) hat mit einem Spannungserhöher angepasst, um mit mindestens einem entsprechenden Abschnitt des genannten Gefäßes (11) nach dem Verschließen des genannten Deckels (12) auf dieses zusammenzuwirken, um mindestens teilweise den genannten Bereich (19) zu brechen und das genannte Behältnis (16) mit dem genannten Gefäß (11) zu verbinden.

2. Der Behälter gemäß Anspruch 1, **dadurch gekennzeichnet, dass** das genannte Gefäß (11) in einem oberen Bereich, ein Innengewinde (13) hat und der genannte Deckel ein komplementär geformtes Außengewinde (14) hat.

3. Der Behälter gemäß einem oder mehreren der vorhergehenden Ansprüche, **dadurch gekennzeichnet, dass** der genannte Gewindeabschnitt des genannten Gefäßes (11) einen größeren Durchmesser hat als der Rest des genannten Gefäßes (11), um eine Stufe und eine ebene ringförmige Fläche (15) zu bilden.

4. Der Behälter gemäß einem oder mehreren der vorhergehenden Ansprüche, **dadurch gekennzeichnet, dass** die genannte Dichtung (17) aus einer Zwischenwand besteht, angepasst zur Hitzeversiegelung auf dem Rand (18) der Öffnung des genannten Deckels (12).

5. Der Behälter gemäß einem oder mehreren der vorhergehenden Ansprüche, **dadurch gekennzeichnet, dass** die genannte Dichtung (17) zwei diametral gegenüberliegende Bereiche (19) hat, die zumindest teilweise von Querschnittsverengungslinien (20) umgeben sind, um Spannungserhöher zu bilden.

6. Der Behälter gemäß einem oder mehreren der vorhergehenden Ansprüche, **dadurch gekennzeichnet, dass** innerhalb des genannten Bereichs (19) sich ein erhöhter Abschnitt (21) davon erstreckt, welcher so angeordnet ist, um mit der genannten ebenen ringförmigen Fläche (15) des genannten Gefäßes (11) nach dem Schließen des genannten Deckels (12) darauf zusammenzuwirken, um zumindest teilweise den genannten Bereich (19) zu brechen und das genannte Behältnis (16) mit dem genannten Gefäß (11) zu verbinden.

7. Der Behälter gemäß einem oder mehreren der vorhergehenden Ansprüche, **dadurch gekennzeichnet, dass** der genannte Bereich (19) laschenförmig ist und sich durch Biegen von dem Rest der genannten Dichtung (17) erhebt, wenn der genannte Deckel (12) aufgedreht wird und der genannte angehobene Abschnitt (21) feststehend bleibt, um auf der genannten ringförmigen Fläche (15) aufzuliegen.

8. Der Behälter gemäß einem oder mehreren der vorhergehenden Ansprüche, **dadurch gekennzeichnet, dass** sich ein Paar von parallel zueinander angeordneten Stiften (22) in der mittigen Position der genannten Dichtung (17) erstrecken.

9. Der Behälter gemäß einem oder mehreren der vorhergehenden Ansprüche, **dadurch gekennzeichnet, dass** das genannte Gefäß (11), der genannte Deckel (12) und die genannte Dichtung (17) aus geformtem Plastikmaterial hergestellt sind.

10. Der Behälter gemäß einem oder mehreren der vorhergehenden Ansprüche, **dadurch gekennzeichnet, dass** das genannte Gefäß (11), der genannte Deckel (12) und die genannte Dichtung (17) aus recyclebarem und/oder bioabbaubarem Polymer hergestellt sind.

## Revendications

1. Contenant (10) pour des échantillons de tissu biologique, comportant un récipient (11) pour déposer un échantillon de tissu et un couvercle (12) adapté pour venir en prise avec ledit récipient (11), ledit couvercle comportant un logement (16) adapté pour contenir un conservateur et un joint (17) rendant étanche l'intérieur dudit logement (16) afin de séparer le contenu de l'extérieur, ledit contenant (10) étant **caractérisé en ce que** ledit joint (17) a au moins une zone (19) avec un concentrateur de contraintes adapté pour interagir avec au moins une partie correspondante dudit récipient (11) lors de la fermeture, sur celui-ci, dudit couvercle (12), pour au moins partiellement casser ladite zone (19) et relier ledit logement (16) audit récipient (11).

2. Récipient selon la revendication 1, **caractérisé en ce que** ledit récipient (11) a, dans une zone supérieure, un filetage intérieur (13) et ledit couvercle a un filetage extérieur (14) formé de manière complémentaire.

3. Contenant selon une ou plusieurs des revendications précédentes, **caractérisé en ce que** ladite partie filetée dudit récipient (11) a un diamètre plus grand que le reste dudit récipient (11) de manière à former un gradin et une surface annulaire plane (15).

4. Contenant selon une ou plusieurs des revendications précédentes, **caractérisé en ce que** ledit joint (17) est constitué d'une séparation adaptée pour un thermoscellage sur le rebord (18) de l'ouverture dudit couvercle (12).

5. Contenant selon une ou plusieurs des revendications précédentes, **caractérisé en ce que** ledit joint (17) a deux zones diamétralement opposées (19) qui sont au moins partiellement entourées par des lignes de réduction de section transversale (20) de manière à former des concentrateurs de contraintes.

6. Conteneur selon une ou plusieurs des revendications précédentes, **caractérisé en ce qu'**à l'intérieur de ladite zone (19), une partie surélevée (21) s'étend à partir de celle-ci, qui est positionnée de manière à interagir avec ladite surface annulaire plane (15) dudit récipient (11) lors de la fermeture, sur celle-ci, dudit couvercle (12), afin d'au moins partiellement casser ladite zone (19) et relier ledit logement (16) audit récipient (11).

7. Contenant selon une ou plusieurs des revendications précédentes, **caractérisé en ce que** ladite zone (19) est en forme de languette et s'élève par flexion par rapport au reste dudit joint (17) lorsque ledit couvercle (12) est vissé dessus et ladite partie surélevée (21) reste fixe pour reposer sur ladite surface annulaire (15) .

8. Contenant selon une ou plusieurs des revendications précédentes, **caractérisé en ce qu'**une paire de broches mutuellement parallèles (22) s'étend dans la position centrale dudit joint (17).

9. Contenant selon une ou plusieurs des revendications précédentes, **caractérisé en ce que** ledit récipient (11), ledit couvercle (12) et ledit joint (17) sont constitués d'une matière plastique moulée.

10. Contenant selon une ou plusieurs des revendications précédentes, **caractérisé en ce que** ledit récipient (11), ledit couvercle (12) et ledit joint (17) sont constitués d'un polymère recyclable et/ou biodégradable.
